# EUROPEAN PATENT APPLICATION

(11) **EP 4 318 408 A1**
(43) Date of publication of application: **07.02.2024**
(21) Application number: 22775642.6
(22) Date of filing: 23.03.2022
(51) Int. Cl.: G06T 19/00, G06T 7/20, G16H 80/00

(54) **INFORMATION PROCESSING SYSTEM, INFORMATION PROCESSING METHOD, AND PROGRAM**

(30) Priority: 23.03.2021 JP 2021048820
(71) Applicant: The University of Tokyo, Bunkyo-ku, Tokyo 113-8654 (JP)
(72) Inventor: NAGAYO Yuri, Tokyo 113-8654 (JP); SAITO Toki, Tokyo 113-8654 (JP); OYAMA Hiroshi, Tokyo 113-8654 (JP)
(74) Representative: Hasegawa, Kan
(86) International application number: PCT/JP2022/013346
(87) International publication number: WO 2022/202860

(57) **Abstract**

[Problem] To provide an information processing system, etc. capable of more accurately transmitting or sharing information on a medical maneuver.

[Solution] One aspect of the invention provides information processing system comprising a control unit configured to execute each of the following steps. A reading step reads reference information on a reference maneuver that is a medical maneuver to be referred to by a user. The reference information includes a three-dimensional position of a track point in the reference maneuver. A first reception step receives a captured image of an external world as viewed by the user. A generation step generates, based on the three-dimensional position of the track point and the captured image, display information for presenting augmented reality regarding the reference maneuver to the user. The augmented reality three-dimensionally reproduces an aspect of a medical instrument used in the reference maneuver or a hand manipulating the medical instrument.

## Description

### TECHNICAL FIELD

The present invention relates to an information processing system, an information processing method, and a program.

### RELATED ART

There are situations where one wishes to share various kinds of information on medical care with others, especially with other doctors. For example, Patent Document 1 discloses a medical support system for sharing patient information as an example of information on medical care.

### PRIOR ART DOCUMENTS

### Patent Document

[Patent Document 1] Japanese Patent Application, Publication No. 2019-109763

### SUMMARY

### Problems to be Solved by Invention

However, although it is possible to share objective information such as text, images, etc. as in the system disclosed in Patent Document 1, it is difficult to communicate or share information on doctors' skill such as movements of hands or medical instruments during surgical operations. This applies, for example, to situations where a skilled doctor wants to demonstrate his/her maneuver to a resident doctor trying to improve his/her maneuver, or where specialists in a certain medical field want to share their respective skills with each other. In other words, in such situations, it is difficult to accurately communicate or share information on these medical maneuvers with others.

In view of the above circumstances, the present invention provides an information processing system, etc. for enabling more accurate communication or sharing of information on a medical maneuver.

### Means for Solving Problems

According to an aspect of the present invention, an information processing system is provided. This information processing system includes a controller. The controller is configured to execute each of following steps. A reading step reads reference information on a reference maneuver that is a medical maneuver to be referred to by a user. The reference information includes a three-dimensional position of a track point in the reference maneuver. A first reception step receives a captured image acquired by capturing an image of an external world as viewed by the user. A generation step generates, based on the three-dimensional position of the track point and the captured image, display information for presenting augmented reality regarding the reference maneuver to the user. The augmented reality three-dimensionally reproduces an aspect of a medical instrument used in the reference maneuver or a hand manipulating the medical instrument.

According to such an aspect, it is possible to realize communication or sharing of information on a medical maneuver with others with improved accuracy and a higher volume of information.

### BRIEF DESCRIPTION OF DRAWINGS

FIG. 1 is a configuration diagram illustrating an information processing system 1 according to the first and second embodiments.
FIG. 2 is a block diagram illustrating a hardware configuration of the information processing apparatus 2 according to the first and second embodiments.
FIG. 3 is a block diagram illustrating a hardware configuration of an augmented reality presentation apparatus 3 according to the first and second embodiments.
FIG. 4 is a block diagram illustrating functions realized by a controller 33, etc. in the augmented reality presentation apparatus 3 according to the first and second embodiments.
FIG. 5 is an activity diagram illustrating a procedure of information processing executed by the information processing apparatus 2 according to the first embodiment.
FIG. 6 is a schematic diagram illustrating an aspect where an image capture apparatus 211 captures an image of a reference maneuver performed by an information provider.
FIG. 7 is an activity diagram illustrating a procedure of information processing executed by the augmented reality presentation apparatus 3 according to the first embodiment.
FIG. 8 is a diagram illustrating an aspect example of a screen 5 of a display unit 34 viewed by a user.
FIG. 9 is a diagram illustrating an aspect example of the screen 5 of the display unit 34 viewed by a user.
FIG. 10 is an activity diagram illustrating a procedure of information processing executed by the information processing system 1 according to the second embodiment.

### DETAILED DESCRIPTION

A description will be given of the embodiments of the present invention with reference to drawings. Various features described in the following embodiments can be combined with each other.

A program for realizing a software described in the present embodiment may be provided as a non-transitory computer-readable memory medium, may be provided to be downloaded via an external server, or may be provided so that the program is activated on an external computer and the program's function is realized on a client terminal (that is, the function is provided by so-called cloud computing).

A term "unit" in the present embodiment may include, for example, a combination of a hardware resource implemented as circuits in a broad sense and information processing of software that can be concretely realized by the hardware resource. Furthermore, various kinds of information are described in the present embodiment, and such information may be represented by, for example, physical values of signal values representing voltage and current, high and low signal values as a set of binary bits consisting of 0 or 1, or quantum superposition (so-called qubits), and communication and computation may be executed on a circuit in a broad sense.

The circuit in a broad sense is a circuit realized by properly combining at least a circuit, circuitry, a processor, a memory, and the like. In other words, a circuit includes an application specific integrated circuit (ASIC), a programmable logic device (e.g., simple programmable logic device (SPLD), a complex programmable logic device (CLPD), field programmable gate array (FPGA), and the like.

### FIRST EMBODIMENT

### 1. Hardware Configuration

This section will describe a hardware configuration according to the first embodiment.

### 1.1 Information Processing System 1

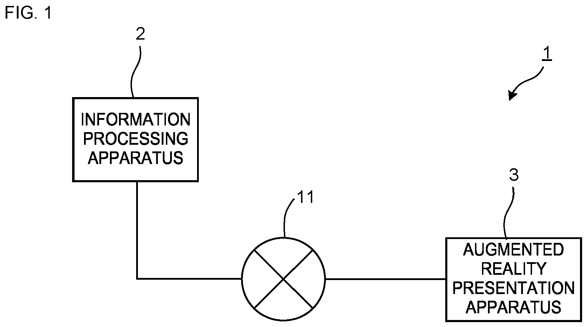

FIG. 1 is a configuration diagram representing an information processing system 1 according to the present embodiment. The information processing system 1 includes an information processing apparatus 2 and an augmented reality presentation apparatus 3, and these are connected via a network 11. These components will be further described. A system exemplified by the information processing system 1 consists of one or more apparatuses or components. Therefore, even the information processing apparatus 2 or the augmented reality presentation apparatus 3 alone can be an example of a system.

### 1.2 Information Processing Apparatus 2

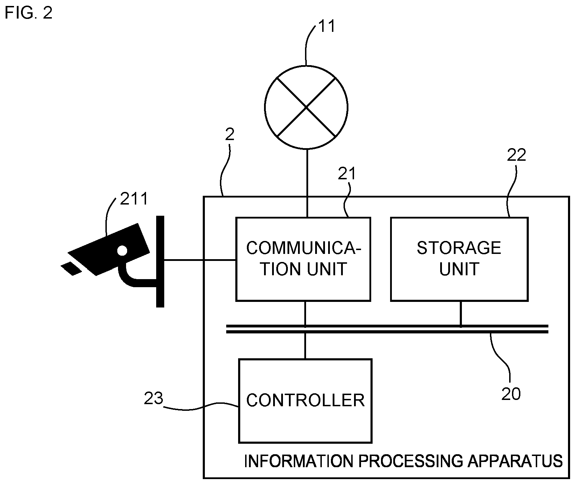

FIG. 2 is a block diagram illustrating a hardware configuration of the information processing apparatus 2 according to the present embodiment. The information processing apparatus 2 includes a communication unit 21, a storage unit 22, and a controller 23, and these components are electrically connected inside the information processing apparatus 2 via a communication bus 20. Each component will be further described.

The communication unit 21 may be wired communication means such as USB, IEEE1394, Thunderbolt (registered trademark), wired LAN network communication, or the like, but may include wireless LAN network communication, mobile communication such as 3G/LTE/5G, Bluetooth (registered trademark) communication, or the like as needed. That is, the communication unit 21 may be implemented as a set of two or more of these communication means.

In the present embodiment, the information processing apparatus 2 is configured to communicate various kinds of information with the augmented reality presentation apparatus 3 by the communication unit 21 via the network 11.

In the present embodiment, the information processing apparatus 2 is connected to an image capture apparatus 211 via the communication unit 21. The image capture apparatus 211 is a so-called vision sensor (camera) configured to capture information on the external world. The image capture apparatus 211 is configured to capture an image of a medical maneuver performed by an information provider for a user. As data of chronologically ordered captured images IM1, a motion image file including the medical maneuver of the information provider may be recorded.

A frame rate of the image capture apparatus 211 is not particularly limited, but may be, for example, 10 fps or higher, may be 30 fps or higher, or may be 60 fps or higher. A higher frame rate allows for higher temporal resolution of below-described reference information IF1.

The storage unit 22 stores above-defined various kinds of information. The storage unit 22 may be implemented, for example, as a storage device such as a solid state drive (SSD) storing various programs, etc. pertaining to the information processing apparatus 2 and executed by the controller 23, or as a memory such as a random access memory (RAM) storing temporarily necessary information (arguments, sequences, etc.) pertaining to program operations. The storage unit 22 may also be a combination thereof.

The controller 23 processes and controls overall operation pertaining to the information processing apparatus 2. The controller 23 is, for example, an unshown central processing unit (CPU). The controller 23 realizes various functions pertaining to the information processing apparatus 2 by reading a predetermined program stored in the storage unit 22. In other words, the controller 23 as an example of hardware is configured to concretely realize information processing of software stored in the storage unit 22.

### 1.3 Augmented Reality Presentation Apparatus 3

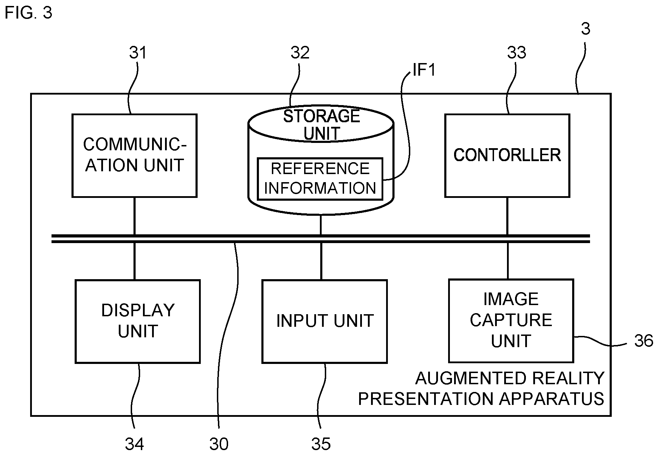

FIG. 3 is a block diagram illustrating a hardware configuration of the augmented reality presentation apparatus 3 according to the present embodiment. The augmented reality presentation apparatus 3 is an apparatus of providing augmented reality to the user. The augmented reality presentation apparatus 3 includes a communication unit 31, a storage unit 32, a controller 33, a display unit 34, an input unit 35, and an image capture unit 36, and these components are electrically connected inside the augmented reality presentation apparatus 3 via a communication bus 30. Each component will be further described below.

The communication unit 31 may be wired communication means such as USB, IEEE 1394, Thunderbolt, wired LAN network communication, and the like, but may include wireless LAN network communication, mobile communication such as 3G/LTE/5G, Bluetooth (registered trademark) communication, or the like as needed. That is, the communication unit 21 may be implemented as a set of two or more of these communication means.

In the present embodiment, the augmented reality presentation apparatus 3 is configured to communicate various kinds of information with the information processing apparatus 2 by the communication unit 31 via the network 11.

The storage unit 32 stores above-defined various kinds of information. The storage unit 32 may be implemented, for example, as a storage device such as a solid state drive (SSD) storing various programs, etc. pertaining to the augmented reality presentation apparatus 3 and executed by the controller 33, or as a memory such as a random access memory (RAM) storing temporarily necessary information (arguments, sequences, etc.) pertaining to program operations. The storage unit 32 may also be a combination thereof.

In particular, the storage unit 32 stores reference information IF1 received from the information processing apparatus 2. In addition to this, the storage unit 32 also stores various programs and the like pertaining to the augmented reality presentation apparatus 3 and executed by the controller 33.

The controller 33 processes and controls overall operation pertaining to the augmented reality presentation apparatus 3. The controller 33 is, for example, an unshown central processing unit (CPU). The controller 33 realizes various functions pertaining to the augmented reality presentation apparatus 3 by reading a predetermined program stored in the storage unit 32. In other words, the controller 33 as an example of hardware concretely realizes information processing of software stored in the storage unit 32, and thereby the controller 33 includes each functional unit described below. These will be described in more detail in the next section. The controller 33 is not limited to being a single controller and may be implemented as two or more controllers 33 for each function or as a combination thereof.

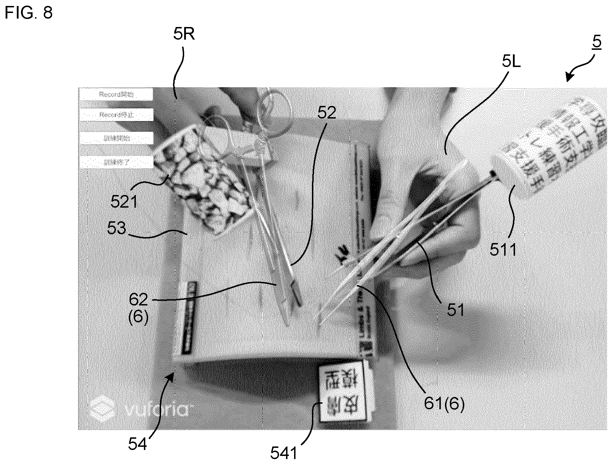

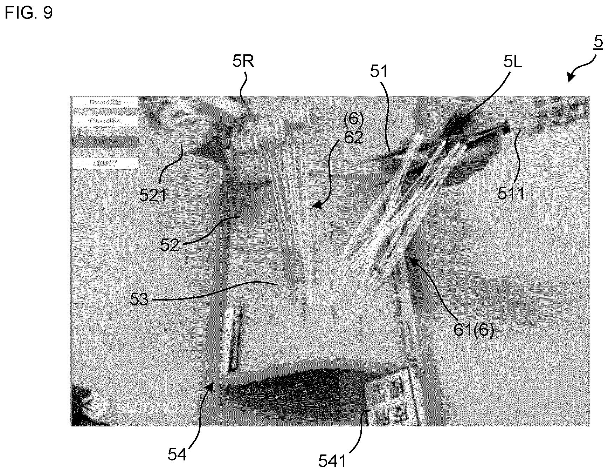

The display unit 34 is configured to display a graphical user interface (GUI) screen 5 that is visible and operable to the user (see FIG. 8 and FIG. 9). The display unit 34 is described here as being included in a housing of the augmented reality presentation apparatus 3. The display unit 34 may be included in a wearable device wearable for the user. In other words, the augmented reality presentation apparatus 3 may be configured as a head-mounted display type apparatus that can be worn by the user in a manner such that the apparatus covers an area around user's eyes. By having such a configuration, the augmented reality presentation apparatus 3 can provide the user with improved immersion. Furthermore, the display unit 34 may be configured to transmit light from the external world in a direction toward the user. Such a configuration allows the user to directly view information from the external world optically, which improves usability.

The input unit 35 is configured to receive operation input made by the user. The input unit 35 is, for example, a switch button provided on the augmented reality presentation apparatus 3. The input is transferred as an instruction signal to the controller 33 via the communication bus 30, and then the controller 33 may execute predetermined control or computation as necessary.

The image capture unit 36 is a so-called vision sensor (camera) configured to capture information on the external world. Here, the image capture unit 36 is described as being included in a housing of the augmented reality presentation apparatus 3. The image capture unit 36 is configured to track a desired track point by capturing, as a captured image IM2, an image of a medical maneuver performed by the user. The captured image IM2 may be implemented as data only temporarily stored for tracking in a memory, or may be implemented to be recorded as a motion image file.

A frame rate of the image capture unit 36 is not particularly limited, but may be, for example, 10 fps or higher, may be 30 fps or higher, or may be 60 fps or higher. A higher frame rate allows for higher temporal resolution of tracking.

### 2. Functional Structure

This section will describe a functional structure according to the present embodiment. As mentioned above, information processing of software stored in the storage unit 32 is concretely realized by the controller 33 as an example of hardware, and thereby may be executed as each functional unit included in the controller 33.

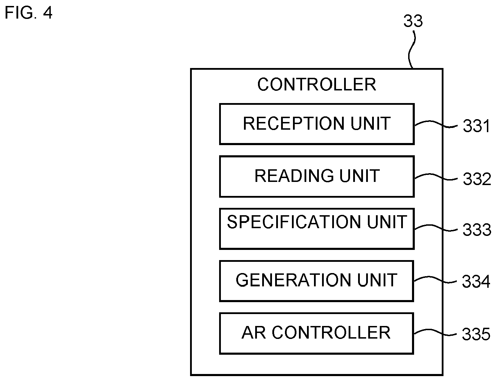

FIG. 4 is a block diagram illustrating functions realized by the controller 33, etc. in the augmented reality presentation apparatus 3 according to the present embodiment. Specifically, the augmented reality presentation apparatus 3 as an example of the information processing system 1 includes a reception unit 331, a reading unit 332, an specification unit 333, a generation unit 334, and an AR controller 335.

The reception unit 331 is configured to receive various kinds of information. For example, the reception unit 331 may receive the reference information IF1 transmitted from the information processing apparatus 2.

The reading unit 332 is configured to read various kinds of information received by the reception unit 331 or stored in advance in the storage unit 32. For example, the reading unit 332 may refer to the reference information IF1 stored in advance in the storage unit 32 so that AR information 6 is presented to the user. This will be described in more detail below.

The specification unit 333 is configured to specify various kinds of information on the basis of the captured image IM 1 and the captured image IM2. For example, the specification unit 333 is configured to specify a surgical field 53 visible to the user and a user maneuver that is a user's medical maneuver, on the basis of the captured image IM2. This will be described in more detail later.

The generation unit 334 is configured to generate various kinds of display information IF2. The display information IF2 may be information itself such as a screen, an image, an icon, a message, etc. generated in an aspect visible to the user, or may be, for example, rendering information for displaying a screen, an image, an icon, a message, etc. on the display unit 34 of the augmented reality presentation apparatus 3. The above-mentioned display unit 34 displays the screen 5 on the basis of the display information IF2 generated by the generation unit 334 (see FIG. 8 and FIG. 9).

The AR controller 335 is configured to control a content or an aspect of augmented reality to be presented to the user. This will be described in more detail later.

### 3. Information Processing Method

This section will describe an information processing method of the above-mentioned information processing system 1. Here, a description will be given of a case where the information provider using the information processing apparatus 2 is a user's attending doctor, and the user is a resident doctor instructed in medical maneuvers by the attending doctor. The augmented reality presentation apparatus 3 is assumed to be the above-mentioned head-mounted display type apparatus worn by the user.

### 3.1 Information Processing by Information Processing Apparatus 2

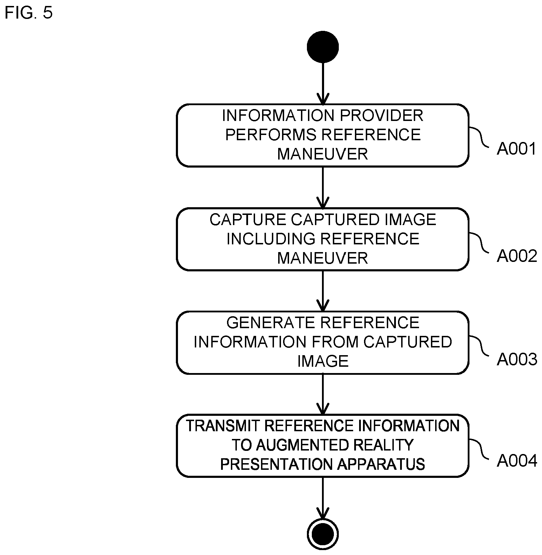

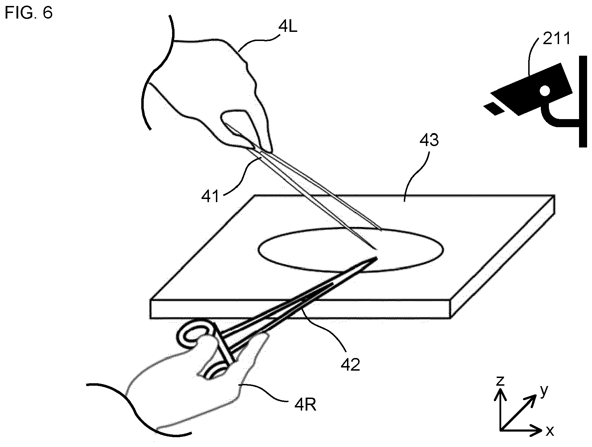

First, a description will be given of information processing by the information processing apparatus 2. FIG. 5 is an activity diagram illustrating a procedure of information processing executed by the information processing apparatus 2 according to the first embodiment. FIG. 6 is a schematic diagram illustrating an aspect of capturing, by the image capture apparatus 211, an image of a reference maneuver performed by the information provider.

First, a procedure of information processing will be outlined with reference to each activity in FIG. 5. First, the information provider performs a reference maneuver, which is a medical maneuver, in front of the image capture apparatus 211 (Activity A001). During this, the image capture apparatus 211 captures an image IM1 including the reference maneuver (Activity A002). Subsequently, the controller 23 in the information processing apparatus 2 reads a predetermined program stored in the storage unit 22 and thereby generates the reference information IF1 acquired by extracting a three-dimensional position of a track point from the captured image IM1 (Activity A003). Finally, via the network 11, the information processing apparatus 2 transmits the reference information IF1 by the communication unit 21 to the augmented reality presentation apparatus 3 used by the user (Activity A004).

Next, a detailed description will be given of the information processing outlined with reference to FIG. 5. In Activity A002, as illustrated in FIG. 6, an image of the reference maneuver of the information provider is captured as the captured image IM1 by the image capture apparatus 211. In the reference maneuver, the information provider uses a forceps 41 as an example of a medical instrument with the left hand 4L and uses a needle holder 42 as an example of a medical instrument with the right hand 4R. Preferably, the image capture apparatus 211 captures a captured image IM 1 such that a three-dimensional position of a predetermined track point can be acquired. Such track point is set for a medical instrument such as the forceps 41, the needle holder 42, etc. Although not illustrated in FIG. 6, the forceps 41, the needle holder 42, etc. may be marked with markers for tracking.

The term "medical instrument" in the present embodiment refers to any instruments concerning medical care. In other words, the medical instrument includes, for example, medical appliances or instruments in accordance with the classification of the Pharmaceutical and Medical Device Act and medical supplies such as needle, suture, etc.

In Activity A003, the controller 23 executes image processing on the captured image IM1 by reading a predetermined program stored in advance. Such image processing extracts the three-dimensional position of at least part of the track points, e.g., the forceps 41 and the needle holder 42, with respect to the surgical field 43. In particular, in acquiring depth information from a viewpoint of the image capture apparatus 211, the depth information may be acquired by estimating the relative sizes of the forceps 41 and the needle holder 42 with respect to the angle of view, may be acquired by the degree of focus, or may be more precisely acquired by stereo vision. The reference information IF1 may be in any form as long as including the above-mentioned three-dimensional position. For example, the reference information may be information consisting of coordinates, or may be a binarized image such that the three-dimensional position can be acquired.

With this configuration, the reference information IF1 generated in Activity A003 includes the three-dimensional position of the track point in the reference maneuver. More specifically, this three-dimensional position may be a relative position in the surgical field 43 of the reference maneuver. According to such an aspect, the subsequent information processing by the augmented reality presentation apparatus 3 can present augmented reality more easily understood by the user. This will be further described.

### 3.2 Information Processing by Augmented Reality Presentation Apparatus 3

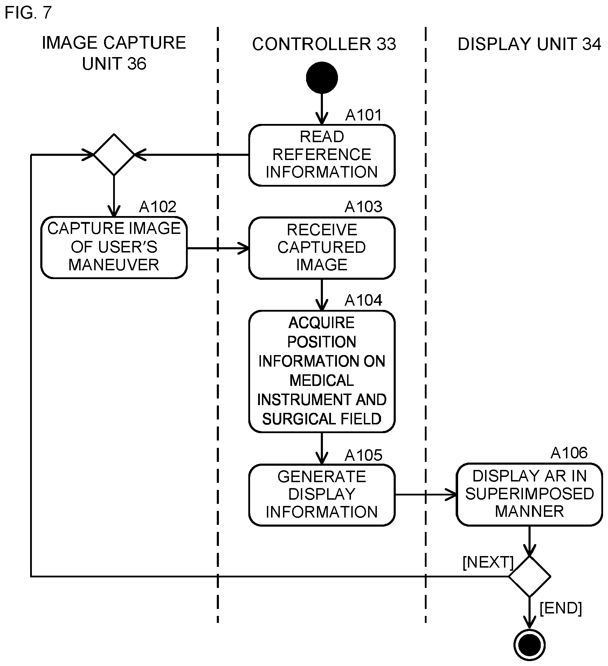

Next, a description will be given of information processing by the augmented reality presentation apparatus 3. FIG. 7 is an activity diagram illustrating a procedure of information processing executed by the augmented reality presentation apparatus 3 according to the present embodiment. FIG. 8 and FIG. 9 are diagrams each illustrating an example of an aspect of the screen 5 of the display unit 34 to be viewed by the user.

First, a procedure of information processing will be outlined with respect to each activity in FIG. 7. First, the reading unit 332 as a functional unit of the controller 33 reads, as a reading step, reference information IF1 on a reference maneuver that is a medical maneuver to be referred to by the user (Activity A101). The reference information IF1 is generated in the information processing apparatus 2 and stored in advance in the storage unit 32 of the augmented reality presentation apparatus 3.

Subsequently, the image capture unit 36 operates with the user wearing the augmented reality presentation apparatus 3 on the user's head and with user's gaze directed toward user's own medical maneuver (hereinafter referred to as user maneuver) so that the user maneuver is within the angle of view of the image capture unit 36. In other words, the image capture unit 36 captures a captured image IM2 including the external world and the user maneuver as viewed by the user (Activity A102).

Subsequently, the reception unit 331 receives, as a first reception step, the captured image IM2 acquired by capturing an image of the external world as viewed by the user (Activity A 103). Specifically, the controller 33 allows a memory as an example of the storage unit 32 to primarily store the captured image IM2.

Subsequently, the specification unit 333 identifies, as a first specification step, a surgical field 53 visible to the user from the captured image IM2 and identifies, as a second specification step, the user maneuver that is a medical maneuver of the user from the captured image IM2. Specifically, the controller 33 identifies, from the captured image IM2, forceps 51 and a needle holder 52 as examples of the medical instrument and the surgical field 53 by reading a predetermined program stored in the storage unit 32, and acquires position information thereon (Activity A104).

Subsequently, the generation unit 334 generates, as a generation step, display information IF2 for presenting AR information 6 (augmented reality) on the reference maneuver to the user on the basis of the three-dimensional position of the track point included in the reference information IF1 and the user maneuver identified from the captured image IM2 (Activity A105). This display information IF2 is, for example, rendering information.

Subsequently, the display unit 34 displays a screen 5 based on the display information IF2 so that the user can view a state where the reference maneuver from the AR information 6 is superimposed on the external world (Activity A106).

The above activities A102 to A106 are repeatedly executed frame by frame, and thereby augmented reality is presented to the user.

In summary, this information processing method includes the following steps. The reading step reads reference information IF1 on a reference maneuver that is a medical maneuver to be referred to by the user. Here, the reference information IF1 includes a three-dimensional position of a track point in the reference maneuver. The first reception step receives a captured image IM2 acquired by capturing an image of the external world as viewed by the user. The generation step generates, based on the three-dimensional position of the track point and on the captured image IM2, display information IF2 for presenting AR information 6 (augmented reality) on the reference maneuver to the user. This augmented reality three-dimensionally reproduce an aspect of a medical instrument used in the reference maneuver or a hand manipulating this medical instrument.

A detailed description will be given of the information processing outlined with reference to FIG. 7. As represented in FIG. 8, the user practices a surgical operation using a model 54 including the visible surgical field 53 with the left hand 5L using the forceps 51 as an example of a medical instrument and with the right hand 5R using the needle holder 52 as an example of a medical instrument.

As represented in FIG. 8, the forceps 51 has a marker 511, the needle holder 52 has a marker 521, and the model 54 has a marker 541. By recognizing these, the specification unit 333 identifies position information on the forceps 51 and the needle holder 52 with respect to the surgical field 53 in the captured image IM2. When it is sufficient to only superimpose information onto the surgical field 53, information on the marker 541 alone may be used. Thereafter, the generation unit 334 generates display information IF2 for presenting, to the user, the AR information 6 (augmented reality) including the medical instrument superimposed on the surgical field 53.

Specifically, the AR information 6 (augmented reality) three-dimensionally reproduces the aspect of the medical instrument used in the reference maneuver. The medical instrument reproduced in the AR information 6 is, for example, AR forceps 61 and an AR needle holder 62 corresponding to the forceps 41 and the needle holder 42 in the reference maneuver. Since, in particular, the AR information 6 is superimposed on the user's surgical field 53, it is possible to transmit more detailed and accurate information from the same perspective as the user's perspective.

As illustrated in FIG. 8, the generation unit 334 may generate the display information IF2 for presenting the AR information 6 (augmented reality) to the user, the display information IF2 being modified according to an aspect of the user maneuver. For example, the AR controller 335 may control progression of the reference maneuver in the AR information 6 (augmented reality) according to the aspect of the user maneuver. It should be noted that in FIG. 8, the positions of the forceps 51 and the needle holder 52 and the positions of the AR forceps 61 and the AR needle holder 62 are close to each other and postures thereof are substantially similar. Specifically, the AR controller 335 executes control to estimate, based on the aspect of the user maneuver acquired from the captured image IM2 by the specification unit 333, a scene to be reproduced of the corresponding reference maneuver. According to such an aspect, the user can check the reference maneuver of the information provider at a pace preferable to the user, which will improve the usability.

Alternatively, as represented in FIG. 9, the augmented reality based on the AR information 6 may reproduce a reference maneuver representing a chronologically future step compared with the current aspect of the user maneuver. In FIG. 9, it should be noted that the positions of the forceps 51 and the needle holder 52 and the positions of the AR forceps 61 and the AR needle holder 62 are different and postures thereof are also different. This is because the AR forceps 61 and AR needle holder 62 are presented in a chronologically future scene compared with the scene assumed from the positions of the forceps 51 and the needle holder 52. According to such an aspect, the user can check the reference maneuver of the information provider while comparing it with the current aspect of the user's maneuver, which will further improve the usability.

The augmented reality based on the AR information 6 may present, to the user, chronological changes in the aspect of the medical instrument, e.g., the AR forceps 61, the AR needle holder 62, etc., with the chronologically changed aspects superimposed on each other. Each of these changed aspects is based on, for example, discretely selected frames. In other words, in FIG. 9, it can be seen that an afterimage-like effect is applied to the AR forceps 61 and the AR needle holder 62. Employing such an effect allows the user to more easily understand the chronological movements of the reference maneuver, which will further improve the usability.

The information processing system 1 according to the first embodiment can realize transmission or sharing of information pertaining to a medical maneuver with others with improved accuracy and a higher volume of information.

### SECOND EMBODIMENT

A description will be given of an information processing system 1 according to the second embodiment. The information processing system 1 according to the second embodiment has the similar hardware configuration and functional configuration to those of the information processing system 1 according to the first embodiment illustrated in FIG. 1 through FIG. 4. Therefore, descriptions thereof will be omitted.

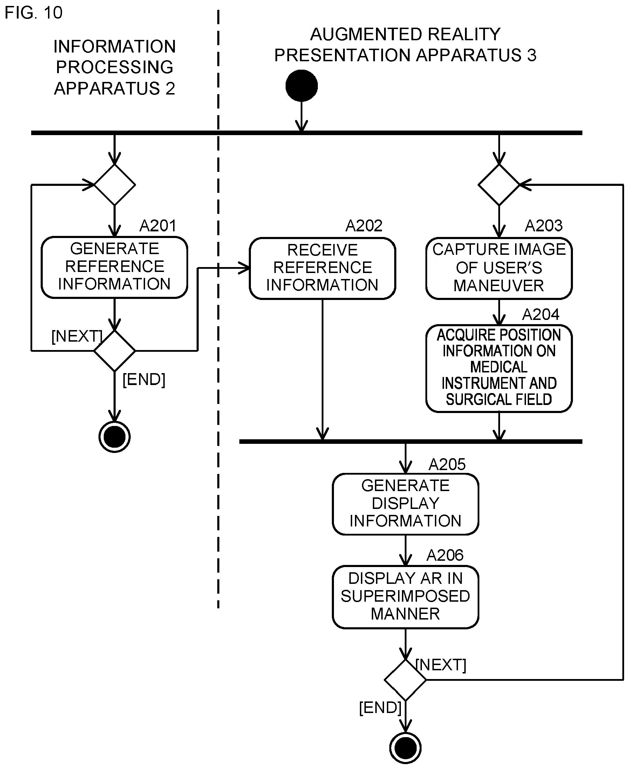

FIG. 10 is an activity diagram illustrating procedure of information processing executed by the information processing system 1 according to the second embodiment.

A description is given of the procedure of the information processing with reference to each activity in FIG. 10. First, as a process on the information processing apparatus 2 side, reference information IF1 is generated for each frame unit of a captured image IM1 captured by the image capture apparatus 211 (Activity A201). The reference information IF1 here may be any information including, for example, position information on information provider's forceps 41 and needle holder 42 with respect to a surgical field 43 for each frame, or may be the captured image IM 1 itself that can be streamed on the augmented reality presentation apparatus 3 side. Subsequently, the communication unit 31 in the augmented reality presentation apparatus 3 continuously receives the reference information IF1 via the network 11 (Activity A202).

Next, as a process on the augmented reality presentation apparatus 3 side, the image capture unit 36 operates with the user wearing the augmented reality presentation apparatus 3 on the user's head and with user's gaze directed toward user's own medical maneuver (user maneuver) so that the user maneuver is within the angle of view of the image capture unit 36. In other words, the image capture unit 36 captures a captured image IM2 including the external world and the user maneuver as viewed by the user (Activity A203). Subsequently, when the reception unit 331 receives the captured image IM2, the specification unit 333 identifies, from the captured image IM2, the forceps 51 and the needle holder 52 as examples of the medical instrument and the surgical field 53 and acquires position information thereon (Activity A204).

Subsequently, as a generation step, the generation unit 334 generates display information IF2 for presenting AR information 6 (augmented reality) on the reference maneuver to the user, on the basis of the three-dimensional position of the track point included in the reference information IF1 and the user maneuver identified from the captured image IM2 (Activity A205). This display information IF2 is, for example, rendering information.

Subsequently, the display unit 34 displays the screen 5 based on the display information IF2 so that the user can view a state where the reference maneuver by the AR information 6 is superimposed on the external world (Activity A206).

Activities A201 and A202 and Activities A203 through A206 are repeatedly executed frame by frame, respectively, and thereby the augmented reality is presented to the user.

To summarize the above, in the second embodiment, the reception unit 331 receives, as the second reception step, reference information IF1 from outside, e.g., from the information processing apparatus 2, via the network 11. The reading unit 332 reads the reference information IF1 received from the information processing apparatus 2. The reception unit 331 may continuously receive, as the second reception step, the reference information IF1 on a reference maneuver in progress by an information provider who is a person different from the user. The reading unit 332 continuously reads the received reference information IF1. The generation unit 334 presents, to the user, the AR information 6 (augmented reality) on the reference maneuver in progress by the information provider.

The information processing system 1 according to the second embodiment can realize, as with the information processing system 1 according to the first embodiment, transmission or sharing of information on a medical maneuver with others with improved accuracy and a higher volume of information. In particular, the information processing system 1 according to the second embodiment can present, to the user, the reference maneuver in progress by the information provider in substantially real time as the AR information 6. This makes it possible, for example, for the information provider to communicate directly with the user while sharing the reference maneuver with the user.

### OTHERS

The information processing system 1 according to the above-mentioned embodiments may include the following aspects.
(1) Although the information provider is described as using the information processing apparatus 2, the information processing apparatus 2 may have the same configuration as the augmented reality presentation apparatus 3. That is, the information provider may use the augmented reality presentation apparatus 3 to capture a captured image IM1 including his/her medical maneuver, and reference information IF1 based on this image may be shared with the augmented reality presentation apparatus 3 used by the user.
(2) The information processing apparatus 2 and the augmented reality presentation apparatus 3 according to the first embodiment may be respectively operated offline, without being connected to each other via the network 11. In such a case, the reference information IF1 generated by the information processing apparatus 2 is shared with the augmented reality presentation apparatus 3 via a communication method other than the network 11, via a storage medium, or the like.
(3) In the above-mentioned embodiments, the reference information IF1 is described as being extracted from the captured image IM 1 by image processing in the information processing apparatus 2, but the reference information IF1 may be the captured image IM1 itself. Furthermore, for example, the specification unit 333 of the augmented reality presentation apparatus 3 may be configured to specify the three-dimensional position of the track point in the reference maneuver on the basis of the captured image IM1, which is the reference information IF1. Thus, the aspects of the reference information IF1 has a wide variety and are not particularly limited.
(4) The display unit 34 of the augmented reality presentation apparatus 3 may not be configured to transmit light from the external world in the direction toward the user. In other words, the display unit 34 may be implemented so that a captured image IM2 as information on the external world captured by the image capture unit 36 is displayed on the display unit 34 as part of the screen 5, and the AR information 6 is superimposed thereon.
(5) The marker 511, the marker 521, and the marker 541 attached to the forceps 51, the needle holder 52, and the model 54 as represented in FIGs. 8 and 9 may not be employed. For example, implementation may be such that the forceps 51, the needle holder52, and the surgical field 53 are recognized based on the shape, color information, or the like instead of the markers and the position information thereon are acquired. Instead of the recognition method, implementation may be such that posture information, optical information such as color information, or the like on the medical instrument may be acquired in addition to the position information. By taking these kinds of information into account, more accurate information sharing can be realized.
(6) The kind of medical instrument is not particularly limited, and further is not limited to an instrument for surgical operations. For example, the medical instrument may include needles and sutures, syringes, catheters, etc., and may also include medical instruments for pinching, sewing, cutting, stripping, etc. More specifically, the medical instrument may include non-toothed forceps, toothed forceps, a Mathieu needle holder, a Hegar needle holder with cemented carbide inserts, a straight micro needle holder with hook, a curved micro needle holder with hook, straight blunt scissors, curved blunt scissors, straight Mayo scissors, curved Mayo scissors, straight Metzenbaum scissors with cemented carbide inserts, non-toothed straight Pean hemostatic forceps, non-toothed curved Pean hemostatic forceps, Alice forceps, straight intestinal forceps, Kelly forceps, etc.
(7) A relationship between the user and the information provider is not limited to the relationship between a resident doctor and an attending doctor, and may be, for example, the relationship between doctors with different specialties. In particular, use of the information processing system 1 according to the second embodiment allows the information provider performing an actual surgical operation to share the scene with the user in substantially real time.
(8) The augmented reality based on the AR information 6 may be configured to be such that a progression speed of the reference maneuver is variable. The user may be able to set a desired progression speed by operating the input unit 35. According to such an aspect, the user can check the reference maneuver of the information provider at a pace preferable to the user, which will improve the usability.
(9) The augmented reality based on the AR information 6 may be configured to be such that the transparency of the medical instrument, e.g., the AR forceps 61 and the AR needle holder 62, is variable. The user may be able to set desired transparency by operating the input unit 35. According to such an aspect, the user can check the reference maneuver of the information provider while adjusting the visibility as preferred for the user, which will improve the usability.
(10) In generating the reference information IF1, instead of the medical instrument used by the information provider, the left hand 4L or the right hand 4R manipulating the medical instrument may be set as the track point. Furthermore, the augmented reality based on the AR information 6 may reproduce, instead of the medical instruments such as the AR forceps 61 and the AR needle holder 62, the hands manipulating these medical instruments. In other words, AR information 6 may be generated that represents hands respectively corresponding to the information provider's left hand 4L and right hand 4R. In such a case, the AR controller 335 may also be able to control the contents of the AR information 6 as necessary.
(11) Various sensors may be used instead of or in combination with the image capture apparatus 211. For example, not only a vision sensor, but also an infrared sensor, a laser rangefinder, LIDAR, etc. may be used as appropriate. Two or more image capture apparatuses may also be used. Such an aspect makes it possible to more accurately acquire three-dimensional information on the external world.
(12) Some of the activities in the activity diagram in FIG. 7 may be omitted. For example, Activity A102, Activity A104, etc. may be omitted.
(13) In the second embodiment, in addition to the substantially real-time information sharing from the information processing apparatus 2 to the augmented reality presentation apparatus 3, substantially real-time information sharing from the augmented reality presentation apparatus 3 to the information processing apparatus 2 may be executed. For example, the user maneuver performed by the user may be able to be further shared to the information provider in substantially real time. When such bi-directional and substantially real-time information sharing is realized, it is possible to realize transmission with more volume of information.
(14) In the above-mentioned embodiment, a description is given of a case where the generation unit 334 generates the display information IF2 for presenting the AR information 6 (augmented reality) to the user, the display information IF2 being modified according to the aspect of the user maneuver, but the display information IF2 is not limited to information on the AR information 6 representing a maneuver as it is. For example, the display information IF2 may include a scoring result acquired by scoring the user maneuver as good or bad, warning, advice, etc. Furthermore, the generation unit 334 may further generate information to be perceived by the user with any sense other than sight. In other words, the generation unit 334 may generate various kinds of information other than the display information IF2, such as sound information, haptics information, olfactory information, etc. Implementation may be such that, when the information provider records the reference maneuver using the information processing apparatus 2, these various kinds of information are recorded at the same time. Such an aspect makes it possible to transmit the information to the user in a more easily understood manner.
(15) The present embodiment may be implemented as a distributable program. This program allows a computer to execute each step in the information processing system 1.
(16) Moreover, the present invention may be provided in each of the following aspects.

The information processing system, wherein: the track point is set on the medical instrument or the hand manipulating the medical instrument, and the three-dimensional position is a relative position with respect to a surgical field of the reference maneuver.

The information processing system, wherein the controller is configured to further execute: a first specification step of specifying, from the captured image, a surgical field visible to the user; and the generation step of generating display information for presenting, to the user, augmented reality where the medical instrument or the hand manipulating the medical instrument is superimposed on the surgical field.

The information processing system, wherein the controller is configured to further execute: a second specification step of specifying a user maneuver from the captured image, the user maneuver being a medical maneuver performed by the user; and the generation step of generating the display information for presenting the augmented reality to the user, the display information being modified according to an aspect of the user maneuver.

The information processing system, wherein, in the augmented reality, progression of the reference maneuver is controlled according to the aspect of the user maneuver.

The information processing system, wherein the augmented reality reproduces the reference maneuver representing a chronologically future step compared with a current aspect of the user maneuver.

The information processing system, wherein the augmented reality is configured to be such that a progression speed of the reference maneuver is variable.

The information processing system, wherein the augmented reality is configured to be such that transparency is variable of the medical instrument or the hand manipulating the medical instrument.

The information processing system, wherein the augmented reality presents, to the user, chronological changes in the aspect of the medical instrument or the hand manipulating the medical instrument, with chronologically changed aspects superimposed on each other.

The information processing system, wherein each of the changed aspects is based on a discretely selected frame.

The information processing system, wherein the reading step reads the reference information stored in advance.

The information processing system, wherein the controller is configured to further execute: a second reception step of receiving the reference information from outside via a network; and the reading step of reading the reference information received from outside.

The information processing system, wherein: the second reception step continuously receives reference information on the reference maneuver in progress by a person different from the user; the reading step continuously reads the received reference information; and the generation step presents augmented reality regarding the reference maneuver in progress to the user.

The information processing system, wherein the generation step further generates information to be perceived by the user with a sense other than sight.

The information processing system, further comprising a display unit configured to: transmit light from the external world in a direction toward the user; and display a screen based on the display information so that the user can view a state where the reference maneuver is superimposed on the external world.

The information processing system, wherein the display unit is included in a wearable device wearable for the user.

An information processing method comprising each step of the information processing system according to any one of claims 1 to 16.

A program configured to allow a computer to execute each step of the information processing system according to any one of claims 1 to 16.

Finally, various embodiments of the present disclosure have been described, but these are presented as examples and are not intended to limit the scope of the disclosure. Novel embodiments can be implemented in various other forms, and various omissions, replacements, and modifications can be made within the scope of the spirit of the disclosure. The embodiments and its modifications are included in the scope and the spirit of the disclosure and are included in the scope of the invention described in claims and the equivalent scope thereof.

### [Description of Sign]

- 1: Information Processing System
- 11: Network
- 2: Information processing apparatus
- 20: Communication bus
- 21: Communication unit
- 211: Image capture apparatus
- 22: Storage unit
- 23: Controller
- 24: Display unit
- 3: Augmented reality presentation apparatus
- 30: Communication bus
- 31: Communication unit
- 32: Storage unit
- 33: Controller
- 331: Reception unit
- 332: Reading unit
- 333: Specification unit
- 334: Generation unit
- 335: AR controller
- 34: Display unit
- 35: Input unit
- 36: Image capture unit
- 4L: Left hand
- 4R: Right hand
- 41: Forceps
- 42: Needle holder
- 43: Surgical field
- 5: Screen
- 5L: Left hand
- 5R: Right hand
- 51: Forceps
- 511: Marker
- 52: Needle holder
- 521: Marker
- 53: Surgical field
- 54: Model
- 541: Marker
- 6: AR information
- 61: AR forceps
- 62: AR needle holder
- IF1: Reference information
- IF2: Display information
- IM1: Captured image
- IM2: Captured image

## Claims

1. An information processing system comprising a controller configured to execute each of following steps including:
a reading step of reading reference information on a reference maneuver that is a medical maneuver to be referred to by a user, the reference information including a three-dimensional position of a track point in the reference maneuver;
a first reception step of receiving a captured image acquired by capturing an image of an external world as viewed by the user; and
a generation step of generating, based on the three-dimensional position of the track point and the captured image, display information for presenting augmented reality regarding the reference maneuver to the user, the augmented reality three-dimensionally reproducing an aspect of a medical instrument used in the reference maneuver or a hand manipulating the medical instrument.

2. The information processing system according to claim 1, wherein:
the track point is set on the medical instrument or the hand manipulating the medical instrument, and
the three-dimensional position is a relative position with respect to a surgical field of the reference maneuver.

3. The information processing system according to claim 1 or 2, wherein the controller is configured to further execute:
a first specification step of specifying, from the captured image, a surgical field visible to the user; and
the generation step of generating display information for presenting, to the user, augmented reality where the medical instrument or the hand manipulating the medical instrument is superimposed on the surgical field.

4. The information processing system according to any one of claims 1 to 3, wherein the controller is configured to further execute:
a second specification step of specifying a user maneuver from the captured image, the user maneuver being a medical maneuver performed by the user; and
the generation step of generating the display information for presenting the augmented reality to the user, the display information being modified according to an aspect of the user maneuver.

5. The information processing system according to claim 4, wherein, in the augmented reality, progression of the reference maneuver is controlled according to the aspect of the user maneuver.

6. The information processing system according to claim 5, wherein the augmented reality reproduces the reference maneuver representing a chronologically future step compared with a current aspect of the user maneuver.

7. The information processing system according to any one of claims 1 to 6, wherein the augmented reality is configured to be such that a progression speed of the reference maneuver is variable.

8. The information processing system according to any one of claims 1 to 7, wherein the augmented reality is configured to be such that transparency is variable of the medical instrument or the hand manipulating the medical instrument.

9. The information processing system according to any one of claims 1 to 8, wherein the augmented reality presents, to the user, chronological changes in the aspect of the medical instrument or the hand manipulating the medical instrument, with chronologically changed aspects superimposed on each other.

10. The information processing system according to claim 9, wherein each of the changed aspects is based on a discretely selected frame.

11. The information processing system according to any one of claims 1 to 10, wherein the reading step reads the reference information stored in advance.

12. The information processing system according to any one of claims 1 to 10, wherein the controller is configured to further execute:
a second reception step of receiving the reference information from outside via a network; and
the reading step of reading the reference information received from outside.

13. The information processing system according to claim 12, wherein:
the second reception step continuously receives reference information on the reference maneuver in progress by a person different from the user;
the reading step continuously reads the received reference information; and
the generation step presents augmented reality regarding the reference maneuver in progress to the user.

14. The information processing system according to any one of claims 1 to 13, wherein the generation step further generates information to be perceived by the user with a sense other than sight.

15. The information processing system according to any one of claims 1 to 14, further comprising a display unit configured to:
transmit light from the external world in a direction toward the user; and
display a screen based on the display information so that the user can view a state where the reference maneuver is superimposed on the external world.

16. The information processing system according to claim 15, wherein the display unit is included in a wearable device wearable for the user.

17. An information processing method comprising each step of the information processing system according to any one of claims 1 to 16.

18. A program configured to allow a computer to execute each step of the information processing system according to any one of claims 1 to 16.
